(19) **Europäisches Patentamt / European Patent Office / Office européen des brevets**

(11) **EP 1 407 777 B1**

(12) **EUROPEAN PATENT SPECIFICATION**

(45) Date of publication and mention of the grant of the patent:
**25.03.2009 Bulletin 2009/13**

(21) Application number: **02733466.3**

(22) Date of filing: **11.06.2002**

(51) Int Cl.:
*A61K 36/185* (2006.01)    *A61P 19/02* (2006.01)

(86) International application number:
**PCT/JP2002/005790**

(87) International publication number:
**WO 2002/102396 (27.12.2002 Gazette 2002/52)**

(54) **HYDRANGEA(E) FOR THE PREVENTION OR TREATMENT OF ARTHRITIS**

HYDRANGEA(E) ZUR PRÄVENTION ODER BEHANDLUNG VON ARTHRITIS

HYDRANGEA(E) POR LA PRÉVENTION OU TRAITEMENT DE L'ARTHRITE

(84) Designated Contracting States:
**AT BE CH CY DE DK ES FI FR GB GR IE IT LI LU MC NL PT SE TR**

(30) Priority: **15.06.2001   JP 2001181947**
**14.03.2002   JP 2002070702**

(43) Date of publication of application:
**14.04.2004   Bulletin 2004/16**

(73) Proprietor: **KYOWA HAKKO KOGYO CO., LTD.**
**Chiyoda-ku,**
**Tokyo 100-8185 (JP)**

(72) Inventors:
- **NAKAGIRI, Ryusuke,**
  **Tsukuba Research Laboratories**
  **Tsukuba-shi,**
  **Ibaraki 305-0841 (JP)**
- **KAMIYA, Toshikazu,**
  **Tsukuba Research Laboratories**
  **Tsukuba-shi,**
  **Ibaraki 305-0841 (JP)**
- **SUDA, Toshio**
  **Sunto-gun,**
  **Shizuoka 411-8731 (JP)**
- **MIKI, Ichiro**
  **Sunto-gun,**
  **Shizuoka 411-8731 (JP)**

(74) Representative: **Vossius & Partner**
**Siebertstraße 4**
**81675 München (DE)**

(56) References cited:
**JP-A- 8 119 829**        **JP-A- 8 176 002**
**JP-A- 10 245 585**       **JP-A- 2000 224 997**

- **PATENT ABSTRACTS OF JAPAN vol. 0185, no. 42 (C-1261), 17 October 1994 (1994-10-17) & JP 6 192114 A (SUNTORY LTD), 12 July 1994 (1994-07-12)**
- **DATABASE WPI Section Ch, Week 200005 Derwent Publications Ltd., London, GB; Class B04, AN 2000-054150 XP002312342 & CN 1 229 658 A (LIU Y) 29 September 1999 (1999-09-29)**
- **DATABASE WPI Section Ch, Week 200042 Derwent Publications Ltd., London, GB; Class B04, AN 2000-476463 XP002312343 & CN 1 242 996 A (LIU B) 2 February 2000 (2000-02-02)**
- **YOUNG HEE KIM ET AL.: 'The production of nitric oxide and TNF-alpha in peritoneal macrophages is inhibited by dichroa febrifuga lour' J. ETHNOPHARMACOLOGY vol. 69, no. 1, 2000, pages 35 - 43, XP002957186**
- **LEVENTHAL L.J. ET AL.: 'Treatment of rheumatoid arthritis with blackcurrant seed oil' BRITISH J. RHEUMATOLOGY vol. 33, no. 9, 1994, pages 847 - 852, XP002957187**
- **SINHA SANGHAMITRA ET AL.: 'Evaluation of anti-inflammatory potential of bergenia ciliata sternb. rhizome extract in rats' J. PHARMACY AND PHARMACOLOGY vol. 53, no. 2, 2001, pages 193 - 196, XP002957188**

**Description**

The present invention relates to an agent for use in preventing or treating arthritis, foods or drinks or feeds for use in preventing or treating arthritis, additives for use in foods and drinks or for feeds for use in preventing or treating arthritis.

JP-A-06/192114 relates to a medicine comprising an extract of Hydrangeae Dulcis Folium as an active ingredient which is stated to have antiinflammatory, antiallergic and inhibitory actions on the decomposition of hyaluronic acid. A food and a cosmetic comprising the medicine are also disclosed.

JP-A-08/176002 describes a cell-anchoring inhibitor, metastasis inhibitor, antiallergic agent and immunosuppressive agent containing, as active ingredients, the plants or extracts therefrom selected from the following: Stellaria neglecta, Aqrimonia pilsoa, Hydrangea macrophylla, Artemigiacapillaris, Euphorbia kansui, Agastache rugosa, Glechoma hederacea, Matricaria chamomila, Euphorbia lathyris, Spirodela ployrhiza, Mentha haplocalyx and Isodom japonicus.

The treatment of rheumatoid arthritis with blackcurrant seed oil is described by L. J. Leventhal et al. in British Journal of Rheumatology, 1994, 33, 847 to 852.

Hydrangea macrophylla Seringe var. thunbergii Makino belonging to the family Saxifragaceae is an allied species of hydrangea and said to have been made through breeding of Hydrangea macrophylla SER var. acuminata in the Edo era. Hydrangeae Dulcis Folium is prepared by fermentation of the harvested leaves and branch ends of Hydrangea macrophylla Seringe var. thunbergii Makino, followed by drying.

Hydrangeae Dulcis Folium or its extract has long been used as a corrective (sweetener) in pills or as a raw material for buccal refrigerants. The aqueous extract of Hydrangeae Dulcis Folium has been used as a sweetener in the form of liquid or powder. The sweetening component is phyllodulcin.

Hydrangeae Dulcis Folium has been known to have an anti-coccidium activity, anti-fungal activity, anti-ulcer activity, anti-allergic activity, hypercholes-terolemia-suppressing activity, anti-periodontal bacteria activity, anti-oxidative activity, and the like (the 2nd Symposium on Medicines and Foods, Summary of Lectures, p.85, 1999). The extract of Hydrangeae Dulcis Folium is known to have a cholagogic effect (Yakugaku Zasshi (Journal of Pharmaceutical Society of Japan), vol. 114, no. 6, 401-413, 1994). In addition, the extract of Hydrangeae Dulcis Folium is also known to show in vitro the effect of inhibiting lipid peroxidation in liver microsomes (Natural Medicines, 49(1), 84-87, 1995).

Saxifraga stolonifera Meerb. belonging to the family Saxifragaceae is a perennial herb growing in colonies on damp rocks in the mountains. The leaves of Saxifraga stolonifera Meerb. have long been known in Japan and China as one of folk medicines, which are believed to exhibit the effect to a skin burn by sticking thereon after crumpling or drying over a fire. The leaves are sometimes supplied for food.

Saxifraga stolonifera Meerb. or its extract has been used as a cosmetic for whitening, a cosmetic for preventing aging of the skin or as a cosmetic for preventing spots or freckles on the face.

Saxifraga stolonifera Meerb. is also known to have a whitening effect (Japanese Patent No.2749218), effect resisting to the mutation induced by ultraviolet light (Japanese Patent No. 1851704), deodorant effect (Japanese Patent No.2015731), testosterone 5α-reductase inhibitory effect (Japanese Published Unexamined Patent Application No. 17365/93), anti-oxidative effect (Japanese Published Unexamined Patent Application No. 46142/98), anti-inflammatory effect by inhibition of histamine release (Japanese Published Unexamined Patent Application No.346911/92), and the like.

However, it has not been known that the plants belonging to the family Saxifragaceae such as Hydrangeae Dulcis Folium and Saxifraga stolonifera Meerb. have an effect of prevention or treatment of arthritis.

An object of the present invention is to provide an agent for preventing or treating arthritis, foods or drinks or feeds for preventing or treating arthritis, additives for foods or for drinks or for feeds for preventing or treating arthritis.

The invention relates to the following (1) to (10).

1. An agent for use in preventing or treating arthritis which comprises a plant of Hydrangea macrophylla Seringe var. Thunbergii Makino or Hydrangeae Dulcis Folium, or an extract thereof as an active ingredient.

2. An agent for preventing or treating arthritis according to item 1, wherein the extract is an alcohol extract of the residue of the extract extracted with an aqueous medium.

3. An agent for preventing or treating arthritis according to item 1 or 2, wherein the arthritis is chronic articular rheumatism.

4. An agent for preventing or treating arthritis according to any of items 1 to 3, which is administered orally.

5. A food or a drink or a feed for use in preventing or treating arthritis which comprises a plant of Hydrangea macrophylla Seringe var. Thunbergii Makino or Hydrangeae Dulcis Folium, or an extract thereof.

6. A food or a drink or a feed according to item 5, wherein the extract is an alcohol extract of the residue of the extract extracted with an aqueous medium.

7. An additive for use in foods or drinks or feeds for use in preventing or treating arthritis, which comprises a plant of Hydrangea macrophylla Seringe var. Thunbergii Makino or Hydrangeae Dulcis Folium, or an extract thereof.

8. An additive for foods or drinks or feeds according to item 7, wherein the extract is an alcohol extract of the residue of the extract extracted with an aqueous medium.

9. Use of a plant of Hydrangea macrophylla Seringe var. Thunbergii Makino or Hydrangeae Dulcis Folium, or an extract thereof for the manufacture of a medicament for preventing or treating arthritis in animals.

10. Use of a plant of Hydrangea macrophylla Seringe var. Thunbergii Makino or Hydrangeae Dulcis Folium, or an extract thereof for the production of an agent for preventing or treating arthritis, a food or drink or feed for preventing or treating arthritis, or an additive for a food or drink or feed for preventing or treating arthritis.

In the present invention, the plants belonging to the family Saxifragaceae mean plants classified under the scientific name Saxifragaceae [Makino's New Illustrated Flora of Japan (40th edition, 1984, Hokuryukan) ; Makino's Illustrated Flora in Color, Sequel (First edition, 1983, Hokuryukan)].

In the present invention, the term "plant" also includes leaves, flowers, branches, stems, fruits, roots, seeds, cultured cells, organs or callus of wild plants, cultivated plants, or plants obtained by culturing such as tissue culture, which are used as such or after being treated physically, chemically or biologically.

The physical or chemical treatment includes drying such as sun-drying, air-drying, and freeze-drying, and disruption. The physically or chemically treated matters include dried matters, freeze-dried matters and disrupted matters. The biological treatment includes fermentation, and the biologically treated matters include fermented matters.

As the plant used in the present invention, a plant of Hydrangea macrophylla Seringe var. Thunbergii Makino or Hydrangeae Dulcis Folium which is obtained by fermentation of Hydrangea macrophylla Seringe var. thunbergii Makino is employed, particularly Hydrangeae Dulcis Folium is preferred.

The extracts of plants include those obtained from the above-described plants by various methods of extraction. The method of extraction includes extraction with various solvents and supercritical fluid extraction. The extract may further be treated by various methods of solid-liquid separation such as sedimentation, cake filtration, clear filtration, centrifugal filtration, centrifugal sedimentation, compression separation and filter press, various concentration methods, various drying methods, methods of making various preparations such as granulation or pulverization, and various purification methods. The purification methods include solvent fractionation, column chromatography and recrystallization.

Particularly, the column chromatography using various carriers such as DIAION HP-20 (Mitsubishi Chemical Corporation) and Sephadex LH-20 (Pharmacia) is preferred.

Examples of the concentration and drying methods are freeze-drying, natural-drying, hot air-drying, air-drying, drying by blowing, spray drying, drying under reduced pressure, sun-drying, vacuum drying, fluidized-bed drying, foam-bed drying, film drying with a drum dryer, ultrasonic drying and electromagnetic ware drying, and the preferred methods are spray drying and freeze-drying.

In the step of extraction and treatment of the extract, an anti-oxidant, preservative, etc. may be added.

As the solvent for extraction, any type of solvent, which can extract a substance having a protective or therapeutical effect for arthritis, may be used. Suitable solvents include aqueous media such as water, an aqueous solution of inorganic salt, buffer solution; monovalent alcohols such as methanol, ethanol, propanol and butanol; polyvalent alcohols such as propylene glycol and glycerol; and organic solvents such as hexane, toluene, petroleum ether, benzene, ethyl acetate, chloroform, dichloromethane, 1,1,2-trichloroethene, dimethylsulfoxide and acetone; and preferred are the aqueous media and alcohols.

Examples of the buffers are phosphate buffer and citrate buffer. The inorganic salt used in the aqueous solution of inorganic salt includes sodium chloride, potassium chloride and calcium chloride.

The preferred alcohols are monovalent alcohols, and a preferred monovalent alcohol is ethanol.

Water may be tap water, distilled water, deionized water, or pure water.

In extracting an active ingredient from the plant containing the active ingredient, it is preferable to use a solvent which is usable for foods or drinks or feeds, for example, water, water-containing ethanol, or anhydrous ethanol, and the like.

These solvent may be used alone or as a mixture. As the mixed solvent, water-containing alcohols are preferred. More preferred are monovalent alcohols, and water-containing ethanol is specifically preferred. The water content is preferably 70% or less, more preferably 40% or less.

As the solvent, supercritical fluid carbon dioxide may also be used.

For extraction, the solvent may be used, for example, in an amount of 0.1 to 10,000 parts by weight preferably 1 to 100 parts by weight for 1 part by weight of a plant. There is no particular limitation for the extraction temperature, but the extraction is preferably carried out at 0 to 100°C, more preferably 20 to 90°C. There is no particular limitation for the extraction time, but it is preferably 1 minute to 1 week, more preferably 30 minutes to 1 day.

The extract containing the active ingredient may be prepared by extraction with various solvents or by supercritical fluid extraction under the condition that the active ingredient can be extracted from the plant containing the active ingredient. The extract thus obtained may further be subjected to extraction with various solvents or by supercritical fluid extraction under the condition that the active ingredient can be extracted. When the active ingredient from the plant cannot be extracted or hardly extracted by extraction with various solvents or by supercritical fluid extraction, the residue obtained after the extraction may further be subjected to extraction with various solvents or by supercritical fluid extraction under the condition that the active ingredient can be extracted.

Specifically, extraction from the plant is preferably carried out by subjecting the plant per se or physically, chemically and/or biologically treated matter of the plant to extraction with an aqueous medium, and then by subjecting the resulting residue (hereinafter referred to as the extraction residue with aqueous medium) to extraction with an alcohol or water-containing alcohol or acetone.

There is no particular limitation for the aqueous medium, but water is preferred. The extraction with an aqueous medium, alcohol, water-containing alcohol or acetone is carried out preferably at a temperature of 0 to 100°C, more preferably 20 to 90°C. There is no particular limitation for the extraction time, but it is preferably 1 minute to 1 week, more preferably 30 minutes to 1 day. It is preferred to the plants after drying. The extraction residue with aqueous medium may be prepared by extracting the plant with an aqueous medium, followed by removal of the aqueous medium through filtration, etc..

There is no particular limitation as to the apparatus used for extraction, and preferred are a vessel designed for efficient extraction, a stirrer, a reflux condenser, a Soxhlet extractor, a homogenizer, a shaker, an ultrasonic generator etc..

There is no limitation as to the arthritis to which the present invention can be applied, and examples include chlamydial arthritis, chronic absorptive arthritis, enteropathic arthritis, gonococcal arthritis, gouty arthritis, Jaccoud's arthritis, juvenile arthritis, Lyme arthritis, ochronotic arthritis, suppurative arthritis, osteoarthritis, shoulder periarthritis, arthritis caused by hyperkinesis, chronic articular rheumatism. The present invention is particularly effective for chronic articular rheumatism.

The following will illustrate agents for use in preventing or treating arthritis, foods or drinks, animal feeds, food additives and feed additives of the present invention.

(1) An agent for preventing or treating arthritis which comprises a plant, of <u>Hydrangea</u> <u>macrophylla</u> Seringe var. Thunbergii Makino or Hydrangeae Dulcis Folium, or an extract thereof as an active ingredient.

An agent for preventing or treating arthritis of the present invention, which comprises the plant of <u>Hydrangea</u> <u>macrophylla</u> Seringe var. Thunbergii Makino or Hydrangeae Dulcis Folium, or an extract thereof as an active ingredient, contains a plant of <u>Hydrangea</u> <u>macrophylla</u> Seringe var. Thunbergii Makino or Hydrangeae Dulcis Folium, or an extract thereof prepared in the manner mentioned above, and it may further contain one or more kinds of pharmacologically acceptable carriers and another active ingredient for prevention or treatment, if necessary. In addition, the agent for preventing or treating arthritis may contain any other ingredient effective for preventing or treating arthritis.

Examples of other ingredients effective for preventing or treating arthritis (hereinafter sometimes simply referred to as other effective ingredients) include boron, calcium, chromium, copper, magnesium, manganese, selenium, silicon, zinc, S-adenosylmethionine, collagen, collagen hydrolyzate, gelatin, gelatin hydrolyzate, bromelain, trypsin, chymotrypsin, papain, rutin, carotenoid, flavonoid, anti-oxidant vitamin, γ-linolenic acid, eicosapentaenoic acid, Boswellia, capsaicin, cat's claw, devil's claw, feverfew, ginger, nettles, niacinamide, shark ointment, turmeric and curcumin. These ingredients may be in pure forms or as a mixture containing them or as an extract.

An agent for preventing or treating arthritis of the present invention can be produced by mixing a plant of <u>Hydrangea</u> <u>macrophylla</u> Seringe var. Thunbergii Makino or Hydrangeae Dulcis Folium, or an extract thereof with one or more pharmacologically acceptable carriers, if required together with another active ingredient, in a way conventionally used in the field of pharmaceutical technology.

It is desirable to administer an agent for preventing or treating arthritis of the present invention through an effective route for preventing or treating arthritis, for example, by oral administration or perenteral administration such as intravenous administration.

The dosage form includes, for example, tablets, powders, granules, pills, suspensions, emulsions, infusions, capsules, syrups, injections, liquid preparations, elixirs, extracts, tinctures, fluid extracts, and the like.

The formulation suitable for oral administration, e.g., extracts, tinctures or fluid extracts, may be prepared by extracting the above plant with water, ethanol or a mixture of water and ethanol, etc., if necessary followed by concentration.

The liquid formulation suitable for oral administration, e.g., syrups, may be prepared using carriers, such as water, a sugar (e.g., sucrose, sorbitol or fructose), a glycol (e.g., polyethylene glycol or propylene glycol), or an oil (e.g., sesame oil, olive oil or soybean oil); an antiseptic (e.g., p-hydroxybenzoate); a preservative such as paraoxybenzoate derivatives (e.g., methyl paraoxybenzoate), sodium benzoate; and a perfume such as strawberry flavor or peppermint.

Tablets, powders, granules, etc., which are suitable for oral administration, may be prepared using a sugar such as lactose, white sugar, glucose, sucrose, mannitol or sorbitol; starch such as potato starch, wheat starch or corn starch; an inorganic substance such as calciumcarbonate, calcium sulf ate, sodium hydrogen carbonate or sodium chloride; an excipient such as crystalline cellulose or plant powder (e.g., licorice powder or gentian powder) ; a disintegrator such as starch, agar, gelatin powder, crystalline cellulose, carmellose sodium, carmellose calcium, calciumcarbonate, sodiumhydrogen carbonate or sodium alginate; a lubricant such as magnesium stearate, talc, hydrogenated vegetable oil, macrogol or silicone oil; a binder such as polyvinyl alcohol, hydroxypropyl cellulose, methylcellulose, ethylcellulose, carmellose, gelatin or starch paste; a surfactant such as fatty acid ester; and/or a plasticizer such as glycerin.

The formulation suitable for non-oral administration preferably comprises a sterilized aqueous preparation containing an active compound which is isotonic to the recipient's blood. For example, for injections, an injectable solution may be prepared using a carrier such as a salt solution, a glucose solution, or a mixture of a salt solution and a glucose solution.

In these non-oral preparations, it is possible to add one or more of supplementary components for the oral preparations mentioned above, selected from carriers, antiseptics, preservatives, flavors, excipients, disintegrators, lubricants, binders, surfactants, and plasticizers.

The dose and frequency of the administration of the agent for preventing or treating arthritis of the present invention depend on the dosage form, age of the patient, body weight, and the symptom and degree of the disease to be treated. For example, in oral administration, a plant of Hydrangea macrophylla Seringe var. Thunbergii Makino or Hydrangeae Dulcis Folium, or an extract thereof may be administered preferably in an amount of 1 mg to 50 g by dry weight, more preferably 5 mg to 10 g, once or several times a day for an adult. In non-oral administration, e.g., intravenous administration, a plant of Hydrangea macrophylla Seringe var. Thunbergii Makino or Hydrangeae Dulcis Folium, or an extract thereof may be administered preferably in an amount of 0.001 to 50 g by dry weight, more preferably 0.01 to 10 g, once or several times a day for an adult.

Arthritis can be prevented by daily administering an agent for arthritis according to the present invention. In the case already attacked by arthritis, it is possible to alleviate or cure the arthritis by daily administering the agent. The agent for preventing or treating arthritis of the present invention is administered usually for a period of 1 week to 10 years, preferably 1 month to 5 years.

The agent for preventing or treating arthritis can be used not only for humans but also for animals other than humans. There is no particular limitation as to the dosage for use in animals. For examples, a plant of Hydrangea macrophylla Seringe var. Thunbergii Makino or Hydrangeae Dulcis Folium, or an extract thereof is administered preferably at a dose of 0.01 to 50 g/kg, and more preferably 0.05 to 10 g/kg by dry weight.

(2) A food or drink or a feed for preventing or treating arthritis which comprises a plant of Hydrangea macrophylla Seringe var. Thunbergii Makino or Hydrangeae Dulcis Folium, or an extract thereof as an active ingredient.

As the food or drink or feed for preventing or treating arthritis of the present invention which comprises a plant of Hydrangea macrophylla Seringe var. Thunbergii Makino or Hydrngeae Dulcis Folium, or an extract thereof or as an active ingredient, is used as such as the food or drink or feed; or alternatively the plant or its extract is used as additives to foods or drinks or feeds. Thus, the food or drink or feed can be used as health food or drink, functional food or drink, etc., for the purpose of preventing or treating arthritis.

(3) A food or drink or feed to which a plant of Hydrangea macrophylla Seringe var. Thunbergii Makino or Hydrangeae Dulcis Folium, or an extract thereof is added.

The food or drink or feed, to which a plant of Hydrangea macrophylla Seringe var. Thunbergii Makino or Hydrangeae Dulcis Folium, or an extract thereof is added, may be produced by adding a plant of Hydrangea macrophylla Seringe var. Thunbergii Makino or Hydrangeae Dulcis Folium, or an extract thereof extracted therefrom in a method as mentioned above to a food or drink or feed which does not contain the plant or its extract.

The food or drink or feed may be produced in a conventional way for production of usual foods or drinks or feeds, except that a plant of Hydrangea macrophylla Seringe var. Thunbergii Makino or Hydrangeae Dulcis Folium, or an extract thereof and if necessary other active ingredients are added.

The food or drink or feed thus obtained may be processed according to a method for processing the usual foods or drinks or feeds, for example, molding or granulation. Examples of the processing method includes granulation methods such as fluid bed granulation, stirring granulation, extrusion granulation, oscillating granulation, gas stream granulation, compression molding granulation, disruption granulation, spray granulation, and jet granulation; coating methods such as pan coating, fluid bed coating and dry coating; and imbibition methods such as puff drying, excess steammethod, foammat method and microwave heating method.

There is no particular limitation for the amount of a plant of Hydrangea macrophylla Seringe var. Thunbergii Makino or Hydrngeae Dulcis Folium, or an extract thereof to be added as far as the foods or drinks or feeds of the present invention for preventing or treating arthritis, and the amount may be, for example, 0.01 to 50% by weight, preferably 0.1 to 10% by weight.

Examples of the foods or drinks prepared by adding a plant of Hydrangea macrophylla Seringe var. Thunbergii Makino or Hydrangeae Dulcis Folium, or an extract thereof include health foods, health drinks, juice, soft drinks, soup, tea, dairy products such as lactic acid bacterium drinks, fermented milk, ice cream, butter, cheese, yogurt, processed milk and skim milk, meat products such as ham, sausage and hamburger, fish paste foods, egg products such as Dashimaki (omelet with stock) and Tamago-dofu (steamed beaten egg with soup stock), confectionary such as cookies, jelly, snacks and chewing gum, bread, noodles, pickles, smoked fish and meat, dry fish, Tsukudani (simmered meat in soy sauce and sugar), and seasonings, to all of which a plant of Hydrangea macrophylla Seringe var. Thunbergii Makino or Hydrangeae Dulcis Folium, or an extract thereof is added.

There is no particular limitation as to the form of the foods or drinks as far as they contain a plant of Hydrangea macrophylla Seringe var. Thunbergii Makino or Hydrangeae Dulcis Folium, or an extract thereof, and examples include powdered foods, sheet-shaped foods, bottled foods, canned foods, retort foods, capsule foods, tablet foods, liquid foods, drinkable preparations, and the like.

To the foods or drinks, if required, it is possible to add one or more additives generally used in foods and drinks, for example, sweetener, coloring agent, preservatives, thickening stabilizer, antioxidant, color-developing agent, bleaching agent, anti-fungal agent, gum base, bitter agents, enzymes, wax, sour agent, seasonings, emulsifier, nutrient supplements, additional materials for preparation, flavors, spice extracts, and the like.

Examples of the sweeteners are aspartame, licorice, stevia, xylose and Momordica grosvenori.

Examples of the coloring agent are carotenoid or turmeric pigment, flavonoid, caramel pigment, oriental gromurell pigment, spirulina pigment, chlorophyll, red sweet potato pigment, red Chinese yam pigment, perilla pigment and blueberry pigment.

Examples of the preservatives are sodium sulfite, benzoic acids, extract of Aralia cordata, extract of Styrax japonica, extract of Artemisia capillaris, sorbic acids, propionic acids, and the like.

Examples of the thickening stabilizers are gums such as gum arabic or xanthan gum, alginic acids, chitin, chitosan, aloe extract, guar gum, hydroxypropylcellulose, casein sodium, corn starch, carboxymethylcelluloses, gelatin, agar, dextrin, methylcellulose, polyvinyl alcohol, microfibrous cellulose, microcrystalline cellulose, seaweed cellulose, sodium polyacrylate, sodium polyphosphate, carrageenan, yeast cell wall, konjak extract, nata de coco and mannan.

Examples of the antioxidants are vitamin C, sodium ethylenediaminetetraacetate, calcium ethylenediaminetetraacetate, erythorbic acid, oryzanol, catechin, quercetin, clove extract, enzyme-treated rutin, apple extract, sesame oil extract, dibutylhydroxytoluene, fennel extract, horseradish extract, dropwort extract, tea extract, Tempeh extract, extract of Houttuynia cordata, tocotrienol, tocopherols, rapeseed oil extract, green coffee extract, sunflower seed, ferulic acid, butylhydroxyanisole, extract of blueberry leaves, propolis extract, hego-ginkgo extract, hesperetin, pepper extract, garden balsam extract, gallic acid, myrica extract, eucalyptus extract and rosemary extract.

An example of the color-developing agent is sodium sulfite.

An example of the bleaching agent is sodium sulfite.

An example of the anti-fungal agent is o-phenylphenol.

Examples of the gum bases are methyl acetylricinoleate, Japanese lacquer wax, ester gum, elemi resin, urucury wax, ozokerite, opopanax resin, kauri gum, carnauba wax, guaiacum resin, gutta katiau, gutta hangkang, gutta-percha, glycerin fatty acid ester, spermaceti, copaiba balsam, copal resin, gum, rice bran wax, sugar cane wax, shellac, jelutong, sucrose fatty acid ester, sorba, sorbitan fatty acid esters, talc, calcium carbonate, dammar resin, chicle, chilte, tunu, low molecular gum, paraffinwax, firbalsam, propylene glycol fatty acid ester, powdered pulp, powdered rice husks, jojoba wax, polyisobutylene, polybutene, microcrystalline wax, mastic, massaranduba chocolate, beeswax and calcium phosphate.

Examples of the bitter agents are isoalpha bitter acid, caffeine, kawaratake extract, cinchona extract, Amur cork extract, gentian extract, spice extract, enzyme-treated naringin, Jamaica quassia extract, theobromine, naringin, bitter ash extract, extract of Artemisia absinthium, isodonis extract, Himematsutake extract, borapet, methylthioadenosine, litchi extract, olive tea, sour orange extract, hop extract and mugwort extract.

Examples of the enzymes are amylase, trypsin, rennet and lactic acid bacteria.

Examples of the wax are Japanese lacquer wax and vegetable wax.

Examples of the sour agents are adipic acid, itaconic acid, citric acids and citrates, succinic acids and succinates, sodium acetate, tartaric acids and tartrates, carbon dioxide, lactic acid, phytic acid, fumaric acid, malic acid and phosphoric

EP 1 407 777 B1

acid.

Examples of the seasonings are amino acids such as asparagine, aspartic acids, glutamic acid, glutamine, alanine, isoleucine, glycine, serine, cystine, tyrosine, leucine, or proline; nucleic acids such as sodium inosinate, sodium uridylate, sodium guanylate, sodium cytidylate, calcium ribonucleotide, or sodium ribonucleotide; organic acids such as citric acid or succinic acid; potassium chloride, sodium solution of low salt content prepared from salt lake water, crude potassium chloride from sea water, whey salt, tripotassium phosphate, dipotassium hydrogenphosphate, potassium dihydrogenphosphate, disodium hydrogenphosphate, sodium dihydrogenphosphate, trisodium phosphate and chlorella extract.

Examples of the emulsifying agents are fatty acid monoglyceride and sorbitan fatty acid ester.

Examples of the nutrient supplements are zinc salts, vitamin C, various amino acids, 5-adenylic acid, iron chloride, hesperidin, various kind of burnt calcium, various kind of unburnt calcium, dibenzoylthiamine, calciumhydroxide, calcium carbonate, thiamine hydrochloride, dunaliella carotene, tocopherol, nicotinic acid, carrot carotene, palm oil carotene, calcium pantothenate, vitamin A, hydroxyproline, calcium dihydrogenpyrophosphate, iron(II) pyrophosphate, iron(III) pyrophosphate, ferritin, heme iron, menaquinone, folic acid and riboflavin.

Examples of the additional materials for preparation are processing aids such as acetone or ion exchange resin, extract of fig leaves, extract of rice straw ash, kaolin, glycerin fatty acid ester, mulberry extract, bone ash, perilla extract, ginger extract, various tannins, Phaffia extract, grape seed extract and ethanol.

Examples of the flavors are those as mentioned above.

Examples of the spice extracts are capsicum extract and garlic extract.

There is no particular limitation as to the intake of the foods and drinks for preventing or treating arthritis which contain a plant of Hydrangea macrophylla Seringe var. Thunbergii Makino or Hydrangeae Dulcis Folium, or an extract thereof to or or the intake of the foods and drinks prepared by adding thereto a plant of Hydrangea macrophylla Seringe var. Thunbergii Makino or Hydrangeae Dulcis Folium, or an extract thereof , however the amount of the plant to be taken is preferably 0.1 g to 50 g, and more preferably 0.5 g to 10 g by dry weight, a day for an adult preferably for a period of one day to one year, more preferably 2 weeks to 3 months. This intake is merely a typical example and the intake can be appropriately adjusted depending on the age, the condition, and body weight of the recipient.

The foods and drinks may be taken once a day or divided in several times. Daily intake of the foods and drinks is effective in prevention of arthritis. Even if the arthritis has already occurred, the daily intake of the foods and drinks would be effective to alleviate or cure arthritis.

The feeds to which has been added a plant of Hydrangea macrophylla Seringe var. Thunbergii Makino or Hydrangeae Dulcis Folium, or an extract thereof can be used as a feed for animals including mammals, birds, reptiles, amphibians and fishes, preferably for mammals. For example, the feeds can be used for pets such as dog, cat or mouse, livestocks such as cattle or pig, poultry such as chicken or turkey, or cultivated fishes such as sea bream or young yellowtail, and preferably for pets or livestocks.

The feeds may be produced by properly mixing feed materials and a plant of Hydrangea macrophylla Seringe var. Thunbergii Makino or Hydrangeae Dulcis Folium, or an extract thereof. The feed materials include cereals, chaff and bran, vegetable oil cakes, animal feed materials, other feed materials and purified products.

Examples of the cereals are milo, wheat, barley, oats, rye, brown rice, buckwheat, foxtail millet, broomcorn mellet, Japanese millet, corn and soybean.

Examples of the chaff and bran are rice bran, defatted rice bran, wheat bran, wheatmiddlings, wheat, wheat germ, barely bran, screening, pellets, corn bran and corn germ.

Examples of the vegetable oil cakes are soybean oil cake, soybean flour, linseed oil cake, cotton seed oil cake, peanut oil cake, safflower oil cake, coconut oil cake, palm oil cake, sesame oil cake, sunflower oil cake, rapeseed oil cake, kapok oil cake and mustard seed oil cake.

Examples of the animal feed materials are fish meal (e.g., northern ocean meal, importedmeal, whole meal and coastal meal), fish soluble, meat meal, meat and bone meal, blood powder, degraded hair, bone meal, processed by-product for livestocks, feather meal, silk-worm pupa, skim milk, casein and dry whey.

Examples of the other feed materials are stems and leaves of plants (e.g., alfalfa, hay cube, alfalfa leaf meal and the powder of false acacia), processed industrial by-products of corn (e.g., corn gluten, meal, corn gluten feed and corn steep liquor), processed starch product, sugar, fermentation industrial products (e. g. , yeast, beer cake, malt root, alcohol cake and soy source cake), agricultural by-products (e.g., processed citrus fruit cake, tofu cake, coffee cake and cocoa cake), and others (e.g. cassava, broad bean, guar meal, sea weed, krill, spirulina, chlorella, minerals).

Examples of the purified products are proteins (e.g., casein and albumin), amino acids, sugars (e.g., starch, cellulose, sucrose and glucose), minerals and vitamins.

There is no particular limitation as to the intake of the feeds for preventing or treating arthritis which contain as an active ingredient a plant of Hydrangea macrophylla Seringe var. Thunbergii Makino or Hydrangeae Dulcis Folium, or an extract thereof or the intake of the feeds prepared by adding a plant of Hydrangea macrophylla Seringe var. Thunbergii Makino or Hydrangeae Dulcis Folium, or an extract thereof , however, it is preferable to take the plant in an amount of 0.01 g to 50 g, more preferably in an amount of 0.05g to 10 g by dry weight/kg by body weight of an animal per day for a period of 1 week to 5 years, more preferably 2 weeks to 2 years. This intake is merely a typical example, and the intake may be appropriately adjusted depending on the species, age, and body weight of the subject animal.

Daily feeding of the animal feeds is effective in prevention of arthritis.

Even if the arthritis has already occurred, the daily feeding of the feeds would be effective to alleviate or cure arthritis.

(4) Additives for foods and drinks or feeds for preventing or treating arthritis which comprise a plant of Hydrangea macrophylla Seringe var. Thunbergii Makino or Hydrangeae Dulcis Folium, or an extract thereof.

The additives for foods and drinks or feeds for preventing or treating arthritis which comprise a plant of Hydrangea macrophylla Seringe var. Thunbergii Makino or Hydrangeae Dulcis Folium, or an extract thereof contain as an active ingredient a plant of Hydrangea macrophylla Seringe var. Thunbergii Makino or Hydrangeae Dulcis Folium, or an extract thereof prepared as mentioned above, and may further contain, if necessary, one or more additives for foods and drinks as listed above, for example, sweeteners, coloring agents, preservatives, thickening stabilizers, antioxidants, color-developing agents, bleaching agents, anti-fungal agents, gum bases, bitter agents, enzymes, wax, sour agents, seasonings, emulsifiers, nutrient supplements, additional materials for preparation, flavors and spice extracts. In addition, the carriers as mentioned above may be added.

There is no limitation as to the intake of the additives for foods and drinks for preventing or treating arthritis, however, it is preferable to take a plant of Hydrangea macrophylla Seringe var. Thunbergii Makino or Hydrangeae Dulcis Folium, or an extract thereof in an amount of 0.1 g to 50 g, more preferably 0.5 g to 10 g by dry weight a day for an adult for a period of 1 week to 10 years, more preferably 1 month to 5 years. This intake is merely a typical example, and the intake may be appropriately adjusted depending on the condition, age, and body weight of the recipient.

There is no limitation as to the amount of the additive to be added to foods and drinks, and for example, the amount is 0.01 to 50% by weight, preferably 0.1 to 10% by weight, though there in no particular limitation therein.

The additives for food and drink may be added to foods and drinks, and the foods or drinks is taken once a day or divided into several times. Daily intake of the additives for foods and drinks is effective in prevention of arthritis. Even if the arthritis has already occurred, the daily intake of the additives for foods and drinks would be effective to alleviate or cure arthritis.

There is no limitation as to the intake of the feed additives for preventing or treat arthritis which contains a plan of Hydrangea macrophylla Seringe var. Thunbergii Makino or Hydrangeae Dulcis Folium, or an extract thereof, however, it is preferable to take the plant or its extract in an amount of 0.01 g to 50 g, more preferably 0.05 to 10g by dry weight per kg by body weight of an animal per day for a period of 1 week to 5 years, more preferably 2 weeks to 2 years. This intake is merely a typical example, and the intake may be appropriately adjusted depending on the species, age, and body weight of the subject animal.

The feed additives may be added to feeds and daily fed to animals to prevent arthritis. Even if the arthritis has already occurred, the daily feeding of the feed additives added to feeds would be effective to cure arthritis.

There is no limitation as to the amount of the feed additive to be added to feeds, however, the amount is for example 0.01 to 50% by weight, preferably 0.1 to 10% by weight.

The following examples will explain the present invention in more detail, but they are not intended to limit the scope of the present invention.

Example 1

Production of freeze-dried powder from a water extract of Hydrangeae Dulcis Folium

Dried powder of Hydrangeae Dulcis Folium (1 kg, Shihira Shoten) was extracted twice with 10 liters of distilled water at room temperature with stirring for 1 hour. The resulting extract was concentrated and freeze-dried to give 200 g of freeze-dried powder of Hydrangeae Dulcis Folium from the water extract.

Example 2

Production of freeze-dried powder from a 60% ethanol-water extract of Hydrangeae Dulcis Folium

Dried powder of Hydrangeae Dulcis Folium (1 kg) was extracted twice with 10 liters of 60% aqueous solution of ethanol at room temperature with stirring for 1 hour. The resulting extract was concentrated and freeze-dried to give 200 g of freeze-dried powder of Hydrangeae Dulcis Folium from the 60% ethanol-water extract.

Example 3

Production of freeze-dried powder of Hydrangeae Dulcis Folium from an acetone extract

Dried powder of Hydrangeae Dulcis Folium (1 kg) was extracted twice with 10 liters of acetone at room temperature with stirring for 1 hour. The resulting extract was concentrated and freeze-dried to give 100 g of freeze-dried powder of Hydrangeae Dulcis Folium from the acetone extract.

Example 4

Production of freeze-dried powder from a hot water extract of Hydrangeae Dulcis Folium

Dried powder of Hydrangeae Dulcis Folium (1 kg) was extracted with 10 liters of boiling distilled water for 30 minutes. The resulting extract was concentrated and freeze-dried to give 130 g of freeze-dried powder of Hydrangeae Dulcis Folium from the hot water extract.

Example 5

Production of freeze-dried powder from an ethanol extract of Hydrangeae Dulcis Folium water-extract residue

Dried powder of Hydrangeae Dulcis Folium (1 kg) was extracted with 20 liters of distilled water at 40°C with stirring until the absorbance of the 1/200 dilution at 313 nm became 0.15. The obtained extract was filtered to remove the filtrate. The resulting extract residue was then extracted with 20 liters of 60% aqueous solution of ethanol at 40°C with stirring until the absorbance of the 1/200 dilution at 313 nm became 0.22. The resulting extract was concentrated and freeze-dried to give 70 g (yield based on dry leaves: 7%) of freeze-dried powder of Hydrangeae Dulcis Folium from the residue of a water extract.

Example 6

Production of freeze-dried powder an acetone extract of Hydrangeae Dulcis Folium water-extract residue

Dried powder of Hydrangeae Dulcis Folium (1 kg) was extracted with 20 liters of distilled water at 40°C with stirring until the absorbance of the 1/200 dilution at 313 nm became 0.15. The obtained extract was filtered to remove the filtrate. The resulting extract residue was then extracted with 20 liters of acetone at 40°C with stirring until the absorbance of the 1/200 dilution at 313 nm became 0.22. The resulting extract was concentrated and freeze-dried to give 60 g (yield based on dry leaves: 6%) of freeze-dried powder of Hydrangeae Dulcis Folium from the water extract residue.

Example 7

Effect of extract of Hydrangeae Dulcis Folium on rats suffering from streptococcal cell wall-induced arthritis

It is known that arthritis is induced in Lewis rats by administering streptococcal cell wall two times [Infection & Immunity, 59(12), 4436 (1991)].

As the first administration of streptococcal cell wall, a peptidoglycan polysaccharide 100P fraction (hereinafter referred to as PG-PS 100) (LEE LABORATORIES), which was diluted with sterilized PBS (-) (9g of sodium chloride; 0.795 gof disodium hydrogenphosphate heptahydrate; 0.144 g of potassium dihydrogenphosphate; dissolved in 1L of distilled water) to 0.6 mg/mL, and destroyed by ultrasonication (US-1 type; made by SND) for 20 minutes, was administered to female Lewis rats (Charles River) of 8 weeks of age at the heel joint of the right hind-limb in a dose of 10μL (6μg of PG-PS 100) per rat. On the other hand, sterilized PBS (-) was administered to the rats into the heel joint of the left hind-limb in a dose of 10μL per rat as a negative control.

At day 21 after the first administration of streptococcal cell wall, as the 2nd administration of streptococcal cell wall, PG-PS 100P, which was similarly diluted with sterilized PBS (-) to 0.2 mg/mL and destroyed by ultrasonication for 2 0 minutes, was intravenously administered to the rats at the tail vein for sensitization in a dose of 500μL/rat (100μL of PG-PS 100). Thus, arthritis was induced in rats.

From 3 days before the first administration of streptococcal cell wall, 1 ml/100 g body weight of 0.5% methylcellulose 400 (Nacalai Tesque) as a control, and 1 ml/100 g body weight of a solution of 30 mg of the ethanol extract from the water-extract residue of Hydrangeae Dulcis Folium (referred to as Ethanol extract of Hydrangeae Dulcis Folium water-extract residue in Table 1) prepared in Example 5 suspended in 0.5% methylcellulose 400 respectively were administered orally to rats 5 times a week. At day 24 after the first administration of streptococcal cell wall, the edema occurring on the joint was measured with a plethysmometer (UNICOM).

In the respective conditions for treatment, 6 rats were used and a change of the edema volume in the joint was calculated from the following formula:

$$\text{Change of the edema volume in the joint} = (A-B)-(C-D)$$

A: volume of the heel joint of the right hind-limb at day 24
B: Volume of the heel joint of the right hind-limb at day 0
C: Volume of the heel joint of the left hind-limb at day 24
D: Volume of the heel joint of the left hind-limb at day 0

Table 1 shows the results.

In Table 1, the figures indicate the change of edema volume in the joint in the respective conditions for treatment by the mean value $\pm$ standard error (n=6).

In Table 1, "Untreated" indicates that no streptococcal cell wall has been administered and "Control" indicates that only 0.5% methylcellulose 400 has been orally administered.

Table 1

| Treatment | Change of the edema volume in joint (ml) | |
| --- | --- | --- |
| | After 0 day | After 24 days |
| Untreated | $0.00 \pm 0.00$ | $0.01 \pm 0.01$ |
| Control | $0.00 \pm 0.00$ | $0.48 \pm 0.11$ |
| Ethanol extract of Hydrangeae Dulcis Folium water-extract residue | $0.00 \pm 0.00$ | $0.16 \pm 0.04$ |

As shown in Table 1, the volume increase in the heel joint of hind-limb, which indicates the degree of joint edema, was inhibited markedly by administration of the ethanol extract of Hydrangeae Dulcis Folium water-extract residue in comparison with that by administration of 0.5% methylcellulose 400 alone (control).

The results indicate that the administration of the ethanol extract of Hydrangeae Dulcis Folium water-extract residue effectively prevents arthritis.

Example 8

Production of an agent containing the ethanol extract of Hydrangeae Dulcis Folium water-extract residue

The following ingredients were mixed to prepare an agent for preventing or treating arthritis.

| | |
| --- | --- |
| Freeze-dried powder prepared in Example 5 | 49 g |
| Pinedex #3 (Matsutani Chemical Industry) | 49 g |
| Ferric pyrophosphate (iron source; Kokusan Chemical Co., Ltd.) | 0.1 g |
| Phoscal EFC (Calcium source; Nikko Fine Products) | 1 g |
| Vitamin mixture (Merck & Co., Inc.) | 1 g |

Example 9

Production of a drink containing the ethanol extract of Hydrangeae Dulcis Folium water-extract residue

Into 180 ml of water was dispersed 20 g of an agent for preventing or treating arthritis prepared in Example 8 to give a drink for prevention or treatment of arthritis.

Example 10

Production of cookies containing the ethanol extract of Hydrangeae Dulcis Folium water-extract residue

Cookies (30 pieces) were prepared from the following ingredients.

| | |
|---|---|
| Soft flour | 100 g |
| Starch | 74 g |
| Water | 14 g |
| Freeze-dried powder prepared in Example 5 | 30 g |
| Baking powder | 2 Tsp. |
| Salt | 2 Tsp. |
| Egg | 1 egg |
| Butter | 80 g |
| Milk | 2 Tbsp. |

Example 11

Production of a feed containing 1% of the freeze-dried powder of Example 1

The following feed was prepared.

| | |
|---|---|
| Sucrose (Kishida Chemical Co., Ltd.) | 20.0 wt% |
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 39.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Freeze-dried powder prepared in Example 1 | 1.0 wt% |

Example 12 (Reference Example)

Production of freeze-dried powder from a water extract of Saxifraga stolonifera Meerb.

Dried powder of Saxifraga stolonifera Meerb. (1 kg, Shihira Shoten) was extracted twice with 10 liters of distilled water at room temperature with stirring for 1 hour. The resulting extract was concentrated and freeze-dried to give 150 g of freeze-dried powder of Saxifraga stolonifera Meerb. as the water extract.

Example 13 (Reference Example)

Production of freeze-dried powder from a 60% ethanol-water extract of Saxifraga stolonifera Meerb.

Dried powder of Saxifraga stolonifera Meerb. (1 kg) was extracted with 20 liters of 60% ethanol-water at 40°C with stirring. The resulting extract was concentrated and freeze-dried to give 315 g of freeze-dried powder of Saxifraga stolonifera Meerb. as the ethanol extract (yield based on dry leaves: 31.5%).

Example 14 (Reference Example)

Production of freeze-dried powder from an acetone extract of Saxifraga stolonifera Meerb.

Dried powder of Saxifraga stolonifera Meerb. (1 kg) was extracted twice with 10 liters of acetone at room temperature with stirring for 1 hour. The resulting extract was concentrated and freeze-dried to give 164 g of freeze-dried powder of Saxifraga stolonifera Meerb. as the acetone extract.

Example 15 (Reference Example)

Production of freeze-dried powder from a hot water extract of Saxifraga stolonifera Meerb.

Dried powder of from Saxifraga stolonifera Meerb. (1 kg) was extracted with 10 liters of boiling distilled water for 30 minutes. The resulting extract was concentrated and freeze-dried to give 100 g of freeze-dried powder of Saxifraga stolonifera Meerb. as the hot water extract.

Example 16 (Reference Example)

Production of freeze-dried powder from an ethanol extract of Saxifraga stolonifera Meerb. water-extract residue

Dried powder of Saxifraga stolonifera Meerb. (1 kg) was extracted with 20 liters of distilled water 40°C with stirring. The extract was filtered to remove the filtrate, and an extract residue was obtained. The residue was extracted with 20 liters of 60% aqueous solution of ethanol at 40°C with stirring. The resulting extract was concentrated and freeze-dried to give 80 g of freeze-dried powder of Saxifraga stolonifera Meerb. as the ethanol extract from the water extraction residue.

Example 17 (Reference Example)

Production of freeze-dried powder from an acetone extract of Saxifraga stolonifera Meerb. water-extract residue

Dried powder of Saxifraga stolonifera Meerb. (1 kg) was extracted with 20 liters of distilledwater at 40°C with stirring. The extract was filtered to remove the filtrate, and an extract residue was obtained. The residue was extracted with 20 liters of acetone at 40°C with stirring. The resulting extract was concentrated and freeze-dried to give 90 gof freeze-driedpowder of Saxifraga stolonifera Meerb. as the acetone extract from the water extraction residue.

Example 18 (Reference Example)

Production of an agent containing the ethanol extract of Saxifraga stolonifera Meerb.

The following ingredients were mixed to prepare an agent for preventing or treating arthritis.

| | |
|---|---|
| Freeze-dried powder prepared in Example 13 | 49 g |
| Pinedex #3 (Matsutani Chemical Industry) | 49 g |
| Ferric pyrophosphate (iron source; Kokusan Chemical Co., Ltd.) | 0.1 g |
| Phoscal EFC (Calcium source; Nikko Fine Products) | 1 g |
| Vitamin mixture (Merck & Co., Inc.) | 1 g |

Example 19 (Reference Example)

Production of a drink containing the ethanol extract of Saxifraga stolonifera Meerb.

The agent for preventing or treating arthritis prepared in Example 18 (20g) was dispersed in 180 ml of water to give a drink for preventing or treating arthritis.

Example 20 (Reference Example)

Production of cookies containing the ethanol extract of Saxifraga stolonifera Meerb.

Cookies (30 pieces) were prepared from the following ingredients.

| | |
|---|---|
| Soft flour | 100 g |
| Starch | 74 g |
| Water | 14 g |
| Freeze-dried powder prepared in Example 13 | 30 g |

(continued)

| | |
|---|---|
| Baking powder | 2 Tsp. |
| Salt | 2 Tsp. |
| Egg | 1 egg |
| Butter | 80 g |
| Milk | 2 Tbsp. |

Example 21 (Reference Example)

Production of a feed containing 1% by weight of the ethanol extract of Saxifraga stolonifera Meerb.

The feed having the following composition was prepared.

| | |
|---|---|
| Sucrose (Kishida Chemical Co., Ltd.) | 20.0 wt% |
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 39.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Freeze-dried powder prepared in Example 13 | 1.0 wt% |

Example 22 (Reference Example)

Production of a feed containing the ethanol extract of Saxifraga stolonifera Meerb.

The feed having the following composition was prepared.

| | |
|---|---|
| CE-2 (Clea Japan, Inc.) | 98.0 wt% |
| Freeze-dried powder prepared in Example 13 | 2.0 wt% |

Example 23 (Reference Example)

Effect of the extract of Saxifraga stolonifera Meerb. on murine arthritis induced by type-2 collagen

In this test, DBA/1J mice (Charles River) were employed, in which arthritis was induced by twice administering type-2 collagen.

As the first administration of type-2 collagen, an equal mixture of type-2 collagen (Collagen Technical Training Institute) and Freund's complete adjuvant (Iatron, Inc.) emulsified with a homogenizer was administered intracutaneously to a male DBA/1J mouse of 6 weeks old at a dose of 100 μl/mouse.

At day 21 after the first administration of type-2 collagen, as the 2nd administration of type-2 collagen, similarly to the first administration, an equal mixture of type-2 collagen and Freund's complete adjuvant emulsified with a homogenizer was administered intracutaneously at a dose of 100 μl/mouse. Thus, arthritis was induced in mice.

Starting on the day of the first administration of type-2 collagen, mice were fed with a feed prepared in Example 22 containing the ethanol extract of Saxifraga stolonifera Meerb. (referred to as "Ethanol extract of Saxifraga stolonifera Meerb." in Table 3). In a control group, mice were fed with powdered feed CE-2 containing nothing. At the days 26, 30, 34, 37 and 42 after the first administration of type-2 collagen, the degree of incidence of arthritis were scored according to the following indices.

Scoring was carried out by applying 0-4 points with respect to one of four paws of the mouse depending on the conditions as shown in Table 2, and then the total score of 4 paws of the mouse, that is, 0 to 16, was recorded. For each condition, 20 mice were subjected to observation.

Table 2

| Condition | Score |
|---|---|
| No swelling observed on digits and hands (feet) (No symptom) | 0 |
| Light swelling observed on one finger or on ankle | 1 |
| Swelling observed on 1 to 3 fingers or on ankle | 2 |
| Swelling observed on 3 to 5 fingers and on ankle | 3 |
| Swelling observed on all fingers and on ankles | 4 |

Table 3 shows the results. The fingers in the table indicate the scores in the respective conditions of treatment by the mean value ± standard error (N = 20). A statistical test for significant difference was carried out by a Student's t-test. In the table, "No treatment" means that the mice have been fed with a commercially available CE-2 and no treatment with type-2 collagen has been made.

Table 3

| Days | Score | | | Significant difference |
|---|---|---|---|---|
| | No treat- | control ment Control | Ethanol Ext.* | |
| 0 | 0.0±0.0 | 0.0±0.0 | 0.0±0.0 | - |
| 26 | 0.0±0.0 | 1.4±0.4 | 1.0±0.4 | - |
| 30 | 0.0±0.0 | 10.4±0.7 | 7.8±0.9 | p = 0.026 |
| 34 | 0.0±0.0 | 12.4±0.5 | 9.0±1.0 | p = 0.004 |
| 37 | 0.0±0.0 | 12.2±0.5 | 9.2±0.9 | p = 0.010 |
| 42 | 0.0±0.0 | 12.0±0.5 | 9.3±1.0 | p = 0.019 |
| * Ethanol extract of Saxifraga stolonifera Meerb. | | | | |

As shown in Table 3, the score of the mice, which were fed with a feed containing 2 wt% of ethanol extract of Saxifraga stolonifera Meerb. was significantly lower than that of the mice (control) which were fed with a powdered feed containing no ethanol extract of Saxifraga stolonifera Meerb., showing that the addition of the ethanol extract of Saxifraga stolonifera Meerb. can inhibit increase of the score of arthritis.

From the above results, it is proved that the incidence of arthritis can be inhibited by previously taking the extract of Saxifraga stolonifera Meerb. before incidence of arthritis.

According to the present invention, it is possible to provide an agent for preventing or treating arthritis, a food or drink or feed for preventing or treating arthritis, an additive to foods or drinks or feeds for preventing or treating arthritis.

**Claims**

1. An agent for use in preventing or treating arthritis which comprises a plant of Hydrangea macrophylla Seringe var. Thunbergii Makino or Hydrangeae Dulcis Folium, or an extract thereof as an active ingredient.

2. An agent for preventing or treating arthritis according to claim 1, wherein the extract is an alcohol extract of the residue of the extract extracted with an aqueous medium.

3. An agent for preventing or treating arthritis according to claim 1 or 2, wherein the arthritis is chronic articular rheumatism.

4. An agent for preventing or treating arthritis according to any of claims 1 to 3, which is administered orally.

5. A food or a drink or a feed for use in preventing or treating arthritis which comprises a plant of Hydrangea macrophylla Seringe var. Thunbergii Makino or Hydrangeae Dulcis Folium, or an extract thereof.

**6.** A food or a drink or a feed according to claim 5, wherein the extract is an alcohol extract of the residue of the extract extracted with an aqueous medium.

**7.** An additive for use in foods or drinks or feeds for use in preventing or treating arthritis, which comprises a plant of <u>Hydrangea</u> <u>macrophylla</u> Seringe var. Thunbergii Makino or Hydrangeae Dulcis Folium, or an extract thereof.

**8.** An additive for foods or drinks or feeds according to claim 7, wherein the extract is an alcohol extract of the residue of the extract extracted with an aqueous medium.

**9.** Use of a plant of <u>Hydrangea</u> <u>macrophylla</u> Seringe var. Thunbergii Makino or Hydrangeae Dulcis Folium, or an extract thereof for the manufacture of a medicament for preventing or treating arthritis in animals.

**10.** Use of a plant of <u>Hydrangea</u> <u>macrophylla</u> Seringe var. Thunbergii Makino or Hydrangeae Dulcis Folium, or an extract thereof for the production of an agent for preventing or treating arthritis, a food or drink or feed for preventing or treating arthritis, or an additive for a food or drink or feed for preventing or treating arthritis.


**Patentansprüche**

**1.** Agens zur Verwendung bei der Vorbeugung oder Behandlung von Arthritis, das eine Pflanze aus <u>Hydrangea</u> <u>macrophylla</u> Seringe var. Thunbergii Makino oder Hydrangeae Dulcis Folium oder ein Extrakt davon als Wirkstoff umfasst.

**2.** Agens zur Vorbeugung oder Behandlung von Arthritis gemäß Anspruch 1, wobei der Extrakt ein Alkoholextrakt des Rückstandes des Extraktes ist, der mit einem wässrigen Medium extrahiert wird.

**3.** Agens zur Vorbeugung oder Behandlung von Arthritis gemäß Anspruch 1 oder 2, wobei die Arthritis chronischer Gelenkrheumatismus ist.

**4.** Agens zur Vorbeugung oder Behandlung von Arthritis gemäß einem der Ansprüche 1 bis 3, das oral verabreicht wird.

**5.** Nahrungsmittel oder Getränk oder Futtermittel zur Verwendung bei der Vorbeugung oder Behandlung von Arthritis, das eine Pflanze aus <u>Hydrangea</u> <u>macrophylla</u> Seringe var. Thunbergii Makino oder Hydrangeae Dulcis Folium oder ein Extrakt davon umfasst.

**6.** Nahrungsmittel oder Getränk oder Futtermittel gemäß Anspruch 5, wobei der Extrakt ein Alkoholextrakt des Rückstandes des Extraktes ist, der mit einem wässrigen Medium extrahiert wird.

**7.** Zusatzstoff zur Verwendung in Nahrungsmitteln oder Getränken oder Futtermitteln zur Verwendung bei der Vorbeugung oder Behandlung von Arthritis, das eine Pflanze aus <u>Hydrangea</u> <u>macrophylla</u> Seringe var. Thunbergii Makino oder Hydrangeae Dulcis Folium oder ein Extrakt davon umfasst.

**8.** Zusatzstoff für Nahrungsmittel oder Getränke oder Futtermittel gemäß Anspruch 7, wobei der Extrakt ein Alkoholextrakt des Rückstandes des Extraktes ist, der mit einem wässrigen Medium extrahiert wird.

**9.** Verwendung einer Pflanze aus <u>Hydrangea</u> <u>macrophylla</u> Seringe var. Thunbergii Makino oder Hydrangeae Dulcis Folium oder eines Extrakts davon zur Herstellung eines Medikaments zur Vorbeugung oder Behandlung von Arthritis bei Tieren.

**10.** Verwendung einer Pflanze aus <u>Hydrangea</u> <u>macrophylla</u> Seringe var. Thunbergii Makino oder Hydrangeae Dulcis Folium oder eines Extrakts davon zur Herstellung eines Agens zur Vorbeugung oder Behandlung von Arthritis, eines Nahrungsmittels oder Getränks oder Futtermittels zur Vorbeugung oder Behandlung von Arthritis oder eines Zusatzstoffs für ein Nahrungsmittel oder Getränk oder Futtermittel zur Vorbeugung oder Behandlung von Arthritis.


**Revendications**

**1.** Agent pour une utilisation dans la prévention ou le traitement de l'arthrite qui comprend une plante d'<u>Hydrangea</u> <u>macrophylla</u> Seringe var. Thunbergii Makino ou d'Hydrangeae Dulcis Folium ou un extrait de celle-ci comme ingrédient actif.

2. Agent pour la prévention ou le traitement de l'arthrite selon la revendication 1, dans lequel l'extrait est un extrait dans un alcool du résidu de l'extrait extrait avec un milieu aqueux.

3. Agent pour la prévention ou le traitement de l'arthrite selon la revendication 1 ou 2, dans lequel l'arthrite est un rhumatisme articulaire chronique.

4. Agent pour la prévention ou le traitement de l'arthrite selon l'une quelconque des revendications 1 à 3, lequel est administré oralement.

5. Aliment ou boisson ou pâture pour une utilisation dans la prévention ou le traitement de l'arthrite qui comprend une plante d'Hydrangea macrophylla Seringe var. Thunbergii Makino ou d'Hydrangeae Dulcis Folium ou un extrait de celle-ci.

6. Aliment ou boisson ou pâture selon la revendication 5, dans lequel l'extrait est un extrait dans un alcool du résidu de l'extrait extrait avec un milieu aqueux.

7. Additif pour une utilisation dans des aliments ou des boissons ou des pâtures pour une utilisation dans la prévention ou le traitement de l'arthrite qui comprend une plante d'Hydrangea macrophylla Seringe var. Thunbergii Makino ou d'Hydrangeae Dulcis Folium ou un extrait de celle-ci.

8. Additif pour des aliments ou des boissons ou des pâtures selon la revendication 7, dans lequel l'extrait est un extrait dans un alcool du résidu de l'extrait extrait avec un milieu aqueux.

9. Utilisation d'une plante d'Hydrangea macrophylla Seringe var. Thunbergii Makino ou d'Hydrangeae Dulcis Folium ou d'un extrait de celle-ci pour la fabrication d'un médicament destiné à la prévention ou au traitement de l'arthrite chez des animaux.

10. Utilisation d'une plante d'Hydrangea macrophylla Seringe var. Thunbergii Makino ou d'Hydrangeae Dulcis Folium ou d'un extrait de celle-ci pour la production d'un agent destiné à la prévention ou au traitement de l'arthrite, d'un aliment ou d'une boisson ou d'une pâture pour la prévention ou le traitement de l'arthrite, ou d'un additif pour un aliment ou une boisson ou une pâture pour la prévention ou le traitement de l'arthrite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6192114 A
- JP 8176002 A
- JP 2749218 B
- JP 1851704 A
- JP 2015731 A
- JP 5017365 A
- JP 10046142 A
- JP 4346911 A

**Non-patent literature cited in the description**

- **L. J. LEVENTHAL et al.** *British Journal of Rheumatology,* 1994, vol. 33, 847-852
- *the 2nd Symposium on Medicines and Foods, Summary of Lectures,* 1999, 85
- **YAKUGAKU ZASSHI.** *Journal of Pharmaceutical Society of Japan,* 1994, vol. 114 (6), 401-413
- *Natural Medicines,* 1995, vol. 49 (1), 84-87
- Makino's New Illustrated Flora of Japan. Hokuryukan, 1984
- Makino's Illustrated Flora in Color, Sequel. Hokuryukan, 1983
- *Infection & Immunity,* 1991, vol. 59 (12), 4436